# EUROPEAN PATENT SPECIFICATION

(11) **EP 1 302 453 B1**
(45) Date of publication and mention of the grant of the patent: **11.03.2009**
(21) Application number: 01941022.4
(22) Date of filing: 13.06.2001
(51) Int. Cl.: C04B 28/34, A61L 27/12, A61K 6/06

(54) **CALCIUM PHOSPHATE CEMENT**
KALZIUMPHOSPHATZEMENT
CIMENT A BASE DE PHOSPHATE DE CALCIUM

(30) Priority: 19.07.2000 JP 2000218964; 28.02.2001 JP 2001054271; 28.02.2001 JP 2001054272; 28.02.2001 JP 2001054273
(43) Date of publication of application: 16.04.2003
(73) Proprietor: HOYA Corporation, Shinjuku-ku Tokyo 161-8525 (JP)
(72) Inventor: HIRANO, Masahiro, Mitsubishi Materials Corp., Chichibu-gun, Saitama 368-8503 (JP); TAKEUCHI, Hiroyasu, Mitsubishi Materials Corp., Tokyo 100-8117 (JP); ASAOKA, Nobuyuki, Mitsubishi Materials Corporation, Chichibu-gun, Saitama 368-8503 (JP)
(74) Representative: Gille Hrabal Struck Neidlein Prop Roos
(86) International application number: PCT/JP2001/004996
(87) International publication number: WO 2002/006179

(56) References cited:
- JP-A- 1 037 445
- JP-A- 2 048 479
- JP-A- 4 314 449
- JP-A- 6 172 007
- JP-A- 6 321 515
- JP-A- 7 008 548
- JP-A- 7 033 489

## Description

### TECHNICAL FIELD

The present invention relates to a calcium phosphate cement that is used, for example, in treatments for reinforcing natural bone in the field of medicine, including oral surgery, and which is capable of forming a high-strength hardened material.

### BACKGROUND ART

Typically, various properties are required of a cement used in treatments for reinforcing natural bone such as: (1) setting properties in which a slurry obtained by adding an aqueous hardening solution to the cement solidifies; (2) hardening properties in which the solidified material hardens in the presence of moisture; (3) sufficient strength of the hardened material in which the hardened material allows movement of the treated reinforced bone; and (4) good absorption replacement in which the hardened material is absorbed by and regenerated into natural bone. Examples of cements having these properties include, for example, a calcium phosphate cement as disclosed in Japanese Unexamined Patent Application, First Publication No. Hei 4-328185, containing 10 to 25 mass% of tetrabasic calcium phosphate, 3 to 10 mass% of dibasic calcium phosphate, and α-tribasic calcium phosphate.

At the same time, the hardened material which is obtained from cements used in treatments for reinforcing natural bone is considered a biologically foreign material. Therefore, it is desirable to obtain the desired amount of strength in the reinforced bone using as little hardened material as possible. Developments have therefore been directed toward improving the strength of the hardened material with the objective of reducing the amount of material employed.

### DISCLOSURE OF THE INVENTION

The present invention was conceived in view of the above-described circumstances and has as an object the provision of a calcium phosphate cement that can be employed in treatments for reinforcing natural bone, and is capable of forming a high-strength hardened material.

The present inventors carried out extensive research directed at improving the strength of the hardened material formed from calcium phosphate cement. Specifically, the present invention was completed with the discovery that by employing a specific calcium phosphate cement it was possible to improve the flow properties of the paste formed by adding an aqueous hardening solution to this calcium phosphate cement, significantly reduce the incorporation of bubbles into the paste, form a compressed hardened material, and remarkably improve the strength of the hardened material formed.

The calcium phosphate cement according to the present invention is formed of a mixed composition including 0.03 to 0.5 mass% magnesium phosphate, 2 to 10 mass% of the product formed from a hydration reaction between dibasic calcium phosphate and α-tribasic calcium phosphate, 3 to 10 mass% dibasic calcium phosphate, and 10 to 25 mass% tetrabasic calcium phosphate, with the remainder comprising α-tribasic calcium phosphate and unavoidable impurities. Further, in the product of a hydration reaction between dibasic calcium phosphate and α-tribasic calcium phosphate, the blending proportion for the dibasic calcium phosphate and the α-tribasic calcium phosphate is such that dibasic calcium phosphate:α-tribasic calcium phosphate = 1:5 to 1:30, preferably dibasic calcium phosphate:α-tribasic calcium phosphate = 1:10 to 1:20. The blending proportion for the dibasic calcium phosphate and the magnesium phosphate when expressed as a mass ratio is dibasic calcium phosphate:magnesium phosphate = 1:0.01 to 1:0.05, and preferably 1:0.02 to 1:0.04.

### PREFERRED EMBODIMENTS OF THE INVENTION

The calcium phosphate cement of the present invention will now be explained more concretely using preferred embodiments thereof.

The calcium phosphate cement of the present invention can be suitably employed in the reinforcement and therapy of vital bone. The calcium phosphate cement of the present invention is not limited to this application solely, however. For example, it may also be used in other applications such as dental cement, plaster alternatives used in dentistry, or construction cement.

The calcium phosphate cement of the present invention is formed of a mixed composition that includes magnesium phosphate in an amount of 0.03 to 0.5 mass%, the product of a hydration reaction between dibasic calcium phosphate and α-tribasic calcium phosphate in an amount of 2 to 10 mass%, dibasic calcium phosphate in an amount of 3 to 10 mass%, and tetrabasic calcium phosphate in an amount of 10 to 25 mass%, with the remainder comprising α-tribasic calcium phosphate, and unavoidable impurities.

The amount of magnesium phosphate included in the calcium phosphate cement is in the range of 0.03 to 0.5 mass% and more preferably in the range of 0.1 to 0.3 mass% with respect to the total mass of the calcium phosphate cement. When the amount of magnesium phosphate included is less than 0.03 mass%, the desired effect of improving the strength of the hardened material tends not to be obtained, while when the amount exceeds 0.5 mass%, the time required for hardening becomes markedly longer.

The amount of dibasic calcium phosphate included in the calcium phosphate cement is in the range of 3 to 10 mass% and more preferably in the range of 4 to 8 mass%, with respect to the total mass of the calcium phosphate cement. When the amount of dibasic calcium phosphate included is less than 3 mass%, the desired effect of promoting setting tends not to be obtained, while when the amount exceeds 10 mass%, hardening of the material is markedly promoted so that workability is impaired.

The tetrabasic calcium phosphate has the effect of promoting absorption replacement in which the hardened material is absorbed and vital bone is regenerated. The amount of tetrabasic calcium phosphate in the calcium phosphate cement is in the range of 10 to 25 mass%, and preferably in the range of 12 to 20 mass%, of the total mass of the calcium phosphate cement. When this amount is less than 10 mass%, the effect of improving absorption replacement tend not to be obtained, while when the amount of tetrabasic calcium phosphate exceeds 25 mass%, the strength of the hardened material decreases.

The product of a hydration reaction between dibasic calcium phosphate and α-tribasic calcium phosphate (hereinafter, referred to as "hydration product") is dispersed in a slurry formed by adding an aqueous solution for hardening to the obtained cement, and serves as a starting point for the hardening reaction. Thus, by adding this hydration product, the hardening reaction occurs at a variety of places in the slurry formed by adding an aqueous hardening solution to the obtained cement, with these hardening reactions progressing simultaneously starting from the hydration product. As a result, the strength of the obtained hardened material is markedly improved. The amount of this hydration product included in the calcium phosphate cement is 2 to 10 mass%, and preferably 3 to 6 mass%, with respect to the total amount of calcium phosphate cement. When the hydration product is included in an amount less than 2 mass%, the desired effect of improving strength tends not to be obtained. In contrast, when the hydration product is included in excess of 10 mass%, the flow properties of the slurry deteriorate and workability is impaired.

The hydration product of the present invention can be formulated by mixing dibasic calcium phosphate and α-tribasic calcium phosphate at mass proportions such that dibasic calcium phosphate:α-tribasic calcium phosphate = 1:5 to 1:30, and preferably dibasic calcium phosphate:α-tribasic calcium phosphate = 1:10 to 1:20, adding water to the obtained mixture to achieve a ratio of mixture:water = 1:0.2 to 1:0.5, and preferably mixture:water = 1:0.3 to 1:0.4, and allowing the hydration reaction to proceed. The resulting material is then ground to a particle diameter in the range of 1 to 300 µm.

The calcium phosphate cement is formulated by the following method, for example. First, magnesium phosphate and dibasic calcium phosphate are mixed at mass proportions such that dibasic calcium phosphate:magnesium phosphate = 1:0.01 to 1:0.05, and preferably 1:0.02 to 1:0.04, and are kneaded together to form a kneaded powder. Next, this kneaded powder and the hydration product are mixed together with the remaining tetrabasic calcium phosphate and α-tribasic calcium phosphate components, to formulate a calcium phosphate cement that is capable of forming a high-strength hardened material.

### TESTS

Next, the calcium phosphate cement of the present invention will be explained more concretely using tests.

### (1) Formulation of α-tribasic calcium phosphate

3 moles of calcium hydroxide were suspended in 10 liters of water. Approximately 0.5 liters of a 40% aqueous phosphoric acid solution formed by diluting 2 moles of phosphoric acid with water were slowly dripped into this suspension while stirring. Once the drop-wise addition was complete, the obtained suspension was allowed to stand at room temperature for one day. A dryer was then employed to dry the suspension for 24 hours at 110°C, to obtain an aggregate. This aggregate was then baked for 3 hours at 1400°C. The baked product was then ground and a screen was used to obtain particles that were 88 µm or less (average particle size: 6.5 µm) in size, to produce a 99.9% pure α-tribasic calcium phosphate.

### (2) Formulation of tetrabasic calcium phosphate

4 moles of calcium hydroxide were suspended in 10 liters of water. Approximately 0.5 liters of a 40% aqueous phosphoric acid solution formed by diluting 2 moles of phosphoric acid with water were slowly dripped into this suspension while stirring. Once the drop-wise addition was complete, the obtained suspension was allowed to stand at room temperature for one day. A dryer was then employed to dry the suspension for 24 hours at 110°C, to obtain an aggregate. Next, the aggregate was pre-baked for 3 hours at 900°C, after which the material was uniformly ground. Baking was then completed by maintaining the material at 1400°C for 3 hours. The obtained baked product was then ground and a screen was used to obtain particles that were 88 µm or less (average particle size: 6.5 µm) in size, to produce a mixed product containing tetrabasic calcium phosphate (content ratio: 90.5%) and hydroxy apatite, an unavoidable impurity. This mixed product was employed as the tetrabasic calcium phosphate.

### (3) Formulation of hydration product

The α-tribasic calcium phosphate formulated in (1) above and a commercially available dibasic calcium phosphate were mixed at the blending proportions shown in Table 1. Water was added to this mixture at a mass ratio of mixture:water = 3:1 and the mixture was hardened. The hardened material was ground to a size of 300 µm or less, to formulate hydration products (a) to (f).

**Table 1**

| Category | | Blending proportion (mass ratio) | |
|---|---|---|---|
| | | Dibasic calcium phosphate | α-tribasic calcium phosphate |
| Hydration product | (a) | 1 | 5 |
| | (b) | 1 | 10 |
| | (c) | 1 | 15 |
| | (d) | 1 | 20 |
| | (e) | 1 | 25 |
| | (f) | 1 | 30 |

Next, the α-tribasic calcium phosphate obtained in the preceding formulations (1) to (3), tetrabasic calcium phosphate, hydration products (a) to (f), and commercially available magnesium phosphate and dibasic calcium phosphate were employed as the starting powders. These starting powders were kneaded for 30 minutes using a kneader at the blending proportions shown in Table 2, to obtain kneaded powders. These kneaded powders were blended with other starting powders at the blending proportions shown in Table 2, and mixed to obtain cements 1 to 16 of the present invention which are the preferred embodiments of this invention, and conventional cements 1 to 5 which are examples of the conventional art.

The strength of the hardened material formed from the thus-obtained cements 1 to 16 of the present invention and conventional cements 1 to 5 was measured and evaluated by the following method. First, an aqueous hardening solution containing 5% chondroitin sodium sulfate, and 15% disodium succinate hexahydrate, with the remainder comprising water, was added to each of the cements at a mass ratio such that cement:aqueous hardening solution = 3:1, and ground to obtain a slurry. This slurry was set, forming a cylindrical aggregate having a diameter of 7 mm and a length of 14 mm. This aggregate was hardened by soaking for five days in an aqueous solution simulating body fluid that contained Na⁺: 142.0 mM, K⁺: 5.0 mM, Mg²⁺: 1.5 mM, Ca²⁺: 2.5 mM, Cl⁻:148.8 mM, HCO³⁻: 4.2 mM, and HPO₄²⁻: 1.0 mM. The compressive strength of the obtained hardened material was then measured and these results are shown in Table 2.

**Table 2**

| Category | | Blending proportion (mass %) | | | | | Compressive strength (MPa) |
|---|---|---|---|---|---|---|---|
| | | Magnesium phosphate | Hydration product | Tetrabasic calcium phosphate | Dibasic calcium phosphate | α-tribasic calcium phosphate + impurities | |
| Cement of the present invention | 1 | 0.2 | a:5 | 18 | 5 | balance | 90 |
| | 2 | 0.2 | b:5 | 18 | 5 | balance | 93 |
| | 3 | 0.2 | c:5 | 18 | 5 | balance | 100 |
| | 4 | 0.2 | d:5 | 18 | 5 | balance | 94 |
| | 5 | 0.2 | e:5 | 18 | 5 | balance | 97 |
| | 6 | 0.2 | f:5 | 18 | 5 | balance | 99 |
| | 7 | 0.03 | c:5 | 18 | 5 | balance | 90 |
| | 8 | 0.1 | c:5 | 18 | 5 | balance | 95 |
| | 9 | 0.3 | c:5 | 18 | 5 | balance | 99 |
| | 10 | 0.5 | c:5 | 18 | 5 | balance | 102 |
| | 11 | 0.2 | c:2 | 18 | 5 | balance | 79 |
| | 12 | 0.2 | c:10 | 18 | 5 | balance | 91 |
| | 13 | 0.2 | c:5 | 10 | 5 | balance | 88 |
| | 14 | 0.2 | c:5 | 25 | 5 | balance | 80 |
| | 15 | 0.2 | c:5 | 18 | 3 | balance | 95 |
| | 16 | 0.2 | c:5 | 18 | 10 | balance | 83 |
| Conventional cement | 1 | - | - | 10 | 5 | balance | 65 |
| | 2 | - | - | 18 | 5 | balance | 62 |
| | 3 | - | - | 25 | 5 | balance | 57 |
| | 4 | - | - | 18 | 3 | balance | 55 |
| | 5 | - | - | 18 | 10 | balance | 53 |

The results shown in Table 2 demonstrate that the hardened material formed from cements 1 to 16 of the present invention, in which magnesium phosphate and the product obtained from a hydration reaction between α-tribasic calcium phosphate and dibasic calcium phosphate were added to a mixed composition, had a higher degree of strength than the hardened material formed using conventional cements 1 to 5 which did not contain the hydration product or the magnesium phosphate.

### INDUSTRIAL APPLICABILITY

The calcium phosphate cement of the present invention enables the formation of a hardened material which has a high strength, and has properties useful in industrial applications such as greatly contributing to an improvement in the strength of treated reinforced bone, and reducing the amount of hardened material employed.

## Claims

1. A calcium phosphate cement, including 0.03 to 0.5 mass% of magnesium phosphate, 2 to 10 mass% of a hydration reaction product of dibasic calcium phosphate and α-tribasic calcium phosphate, 3 to 10 mass% of dibasic calcium phosphate, and 10 to 25 mass% of tetrabasic calcium phosphate, with the remainder being α-tribasic calcium phosphate and unavoidable impurities.

2. The calcium phosphate cement according to claim 1, in which the blending proportion of the dibasic calcium phosphate and the α-tribasic calcium phosphate in said hydration reaction product of dibasic calcium phosphate and α-tribasic calcium phosphate, when expressed as a mass ratio, is such that dibasic calcium phosphate:α-tribasic calcium phosphate = 1:5 to 1:30.

3. The calcium phosphate cement according to claim 1, in which the blending proportion of the dibasic calcium phosphate and the α-tribasic calcium phosphate in said hydration reaction product of the dibasic calcium phosphate and α-tribasic calcium phosphate, when expressed as a mass ratio, is such that dibasic calcium phosphate:α-tribasic calcium phosphate = 1:10 to 1:20.

4. The calcium phosphate cement according to any one of claims 1 to 3, in which the blending proportion of the dibasic calcium phosphate and the magnesium phosphate, when expressed as a mass ratio, is such that dibasic calcium phosphate:magnesium phosphate = 1:0.01 to 1:0.05.

5. The calcium phosphate cement according to any one of claims 1 to 3, in which the blending proportion of the dibasic calcium phosphate and the magnesium phosphate, when expressed as a mass ratio, is such that dibasic calcium phosphate:magnesium phosphate = 1:0.02 to 1:0.04.

## Patentansprüche

1. Calciumphosphatzement, der 0,03 bis 0,5 Ges.-% Magnesiumphosphat, 2 bis 10 Gew.-% eines Hydratationsreaktionsprodukts von dibasischem Calciumphosphat und α-tribasischem Calciumphosphat, 3 bis 10 Gew.-% dibasisches Calciumphosphat, und 10 bis 25 Gew.-% tetrabasisches Calciumphosphat beinhaltet, wobei der Rest α-tribasisches Calciumphosphat und unvermeidbare Verunreinigungen sind.

2. Calciumphosphatzement nach Anspruch 1, wobei das Mischungsverhältnis von dibasischem Calciumphosphat und α-tribasischem Calciumphosphat im Hydratationsreaktionsprodukt von dibasischem Calciumphosphat und αtribasischem Calciumphosphat, ausgedrückt als Gewichtsverhältnis, so ist, dass dibasisches Calciumphosphat: α-tribasisches Calciumphosphat = 1:5 bis 1:30.

3. Calciumphosphatzement nach Anspruch 1, wobei das Mischungsverhältnis von dibasischem Calciumphosphat und α-tribasischem Calciumphosphat im Hydratationsreaktionsprodukt von dibasischem Calciumphosphat und αtribasischem Calciumphosphat, ausgedrückt als Gewichtsverhältnis, so ist, dass dibasisches Calciumphosphatm: α-tribasisches Calciumphosphat = 1:10 bis 1:20.

4. Calciumphosphatzement nach einem der Ansprüche 1 bis 3, wobei das Mischungsverhältnis von dibasischem Calciumphosphat und Magnesiumphosphat, ausgedrückt als Gewichtsverhältnis, so ist, dass dibasisches Calciumphosphat: Magnesiumphosphat = 1:0,01 bis 1:0,05.

5. Calciumphosphatzement nach einem der Ansprüche 1 bis 3, wobei das Mischungsverhältnis von dibasischem Calciumphosphat und Magnesiumphosphat, ausgedrückt als Gewichtsverhältnis, so ist, dass dibasisches Calciumphosphat : Magnesiumphosphat = 1:0,02 bis 1:0,04.

## Revendications

1. Ciment à base de phosphate de calcium, comprenant 0,03 à 0,5% en poids de phosphate de magnésium, 2 à 10% en poids d'un produit de réaction d'hydratation du phosphate de calcium dibasique et du phosphate de calcium α-tribasique, 3 à 10% en poids de phosphate de calcium dibasique, et 10 à 25% en poids de phosphate de calcium tétrabasique, le reste étant du phosphate de calcium α-tribasique et des impuretés inévitables.

2. Ciment à base de phosphate de calcium selon la revendication 1, dans lequel la proportion de mélange de phosphate de calcium dibasique et de phosphate de calcium α-tribasique dans ledit produit de réaction d'hydratation du phosphate de calcium dibasique et du phosphate de calcium α-tribasique, exprimée en tant que rapport pondéral, est telle que le rapport phosphate de calcium dibasique:phosphate de calcium α-tribasique est égal à 1:5 à 1:30.

3. Ciment à base de phosphate de calcium selon la revendication 1, dans lequel la proportion de mélange de phosphate de calcium dibasique et de phosphate de calcium α-tribasique dans ledit produit de réaction d'hydratation du phosphate de calcium dibasique et du phosphate de calcium α-tribasique, exprimée en tant que rapport pondéral, est telle que le rapport phosphate de calcium dibasique:phosphate de calcium α-tribasique est égal à 1:10 à 1:20.

4. Ciment à base de phosphate de calcium selon l'une quelconque des revendications 1 à 3, dans lequel la proportion de mélange de phosphate de calcium dibasique et de phosphate de magnésium, exprimée en tant que rapport pondéral, est telle que le rapport phosphate de calcium dibasique:phosphate de magnésium est égal à 1:0,01 à 1:0,05.

5. Ciment à base de phosphate de calcium selon l'une quelconque des revendications 1 à 3, dans lequel la proportion de mélange de phosphate de calcium dibasique et de phosphate de magnésium, exprimée en tant que rapport pondéral, est telle que le rapport phosphate de calcium dibasique:phosphate de magnésium est égal à 1:0,02 à 1:0,04.
